Europäisches Patentamt

European Patent Office

Office européen des brevets

(19)

(11) Numéro de publication: **0 365 439**
**A1**

(12) # DEMANDE DE BREVET EUROPEEN

(21) Numéro de dépôt: **89420378.5**

(22) Date de dépôt: **09.10.89**

(51) Int. Cl.⁵: **C08J 3/03 , C08L 83/04 , C08K 5/54 , C09D 183/04**

(30) Priorité: **11.10.88 FR 8813617**

(43) Date de publication de la demande:
**25.04.90 Bulletin 90/17**

(84) Etats contractants désignés:
**AT BE CH DE ES FR GB GR IT LI LU NL SE**

(71) Demandeur: **RHONE POULENC CHIMIE**
**25 Quai Paul Doumer**
**F-92408 Courbevoie Cédex(FR)**

(72) Inventeur: **Feder, Michel**
**35, rue du Séminaire Zillisheim**
**F-68720 Illfurth(FR)**
Inventeur: **Frances, Jean-Marc**
**9, avenue Condorcet**
**F-69100 Villeurbanne(FR)**

(74) Mandataire: **Seugnet, Jean Louis et al**
**RHONE-POULENC CHIMIE Service Brevets**
**Chimie Centre de Recherches des Carrières**
**B.P. 62**
**F-69192 Saint-Fons Cédex(FR)**

(54) **Dispersion aqueuse de silicone à base de siliconate réticulant en un élastomère par élimination de l'eau.**

(57) La présente invention concerne une dispersion aqueuse de silicone, réticulant en un élastomère par élimination de l'eau comportant :
- 100 parties d'une émulsion (A) du type huile dans eau d'un $\alpha,\omega$-(dihydroxy)polydiorganosiloxane stabilisée par un tensio-actif anionique et/ou non-ionique,
- 0,1 à 15 parties d'un organosiliconate,
- 5 5 à 250 parties d'un charge minérale non siliceuse,
ladite émulsion ayant un pH supérieur à 7 et une teneur en extraits secs d'au moins 40 %.
  Utilisation des émulsions notamment pour la réalisation de joints élastomère silicone, en particulier pour le bâtiment et le revêtement d'objets en contact avec des aliments.

EP 0 365 439 A1

# DISPERSION AQUEUSE DE SILICONE A BASE DE SILICONATE RETICULANT EN UN ELASTOMERE PAR ELIMINATION DE L'EAU

La présente invention concerne une dispersion aqueuse de silicone à base de siliconate pouvant réticuler en un élastomère par élimination de l'eau.

Le brevet US-A-2 891 920 décrit un procédé de polymérisation en émulsion de polydiorganosiloxane en utilisant un catalyseur acide ou basique en présence d'agents tensio-actifs anioniques, cationiques ou non-ioniques. Ce brevet enseigne que les émulsions obtenues sont stables au stockage et sont utiles, après addition de charges, pour faire des peintures donnant un revêtement continu par élimination de l'eau.

Le brevet US-A-3 294 725 décrit en particulier l'utilisation d'acide dodécylbenzène-sulfonique pour polymériser en émulsion de polydiorganosiloxanes. Ce brevet enseigne que pour obtenir des émulsions stables, il est souhaitable de régler le pH de ces émulsions à une valeur de 7 environ. Ce brevet enseigne que l'on peut obtenir à partir de ces émulsions neutralisées, auxquelles on a ajouté de la silice colloïdale et un polyalcoxysilane, un revêtement élastomère.

L'enseignement du brevet US-A-3 360 491 est similaire à celui de US-A-3 294 725, sauf que l'acide dodécylbenzène sulfonique est remplacé par l'hydrogénolaurylsulfate.

Le brevet US-A-3 697 469 décrit un procédé particulier de polymérisation en émulsion de polydiorganosiloxanes et indique la possibilité d'ajouter à cette émulsion de la silice colloïdale et un sel d'étain en vue d'obtenir un revêtement élastomère par évaporation d'eau.

Le brevet français FR-A-2 1100 358 décrit une émulsion silicone à pH compris entre 6,5 et 9, réticulant en un élastomère conducteur de l'électricité après évaporation de l'eau par incorporation de noir de carbone. L'émulsion, comportant en outre un sel d'étain et un polyalcoxysilane, n'est pas stable au stockage et doit être conservée en deux emballages distincts (émulsion bicomposante).

Les brevets américains US-A-4 221 688, US-A-4 244 849 et français FR-A-2 463 163 décrivent des émulsions silicone stables au stockage et comportant :
- une émulsion d'un polymère $\alpha,\omega$-(dihydroxy)polydiorganosiloxane stabilisée anioniquement,
  - une charge siliceuse,
  - un sel d'étain,
  - éventuellement une charge non renforçante.

La charge siliceuse peut être une silice colloïdale (US-A-4 221 688), du silicate de soude (US-A-4 244 849), ou une silice amorphe en poudre (FR-A-2 463 163).

Par rapport aux émulsions (dispersions) aqueuses connues de l'art antérieur, ces trois brevets enseignent d'une part que pour obtenir une émulsion monocomposante stable au stockage il faut conserver l'émulsion à un pH alcalin supérieur à 8,5 ou 9, de préférence supérieur à 10, et, d'autre part, incorporer à l'émulsion un sel d'étain pour raccourcir à quelques jours l'étape de maturation de l'émulsion nécessaire à l'obtention d'une dispersion capable de réticuler.

Le brevet US-A-3 355 406 enseigne un latex de silicone constitué d'un $\alpha,\omega$-(dihydroxy)-polydiorganosiloxane, de préférence préparé par polymérisation en émulsion et d'une résine silsesquioxane constituée de motifs $RSiO_{1,5}$ (R reste hydrocarboné). Le latex peut comporter en outre un catalyseur métallique de durcissement et un alkyltrialcoxysilane.

Dans le brevet US-A-4 554 187, la résine silicone associée à l'$\alpha,\omega$-(dihydroxy)polydiorganosiloxane est une résine réactive de faible poids moléculaire présentant des groupes alcoxy ou acyloxy.

Dans la demande de brevet EP-A-266 729 la résine silicone, associée à l'$\alpha,\omega$-(dihydroxy)-polydiorganosiloxane et au catalyseur de durcissement, est un siliconate.

A ce siliconate peut être associé une résine silicone comportant jusqu'à 10% en poids de groupe hydroxyle.

Des latex silicones sans catalyseur figurent dans les brevets EP-A-166 396 et EP-A-169 ainsi que dans EP-A-277 740, mais ils ne comportent pas de résines silicones.

L'examen de l'art antérieur montre que la plupart des dispersions aqueuses de silicones comportant un catalyseur de durcissement qui est un sel métallique, en général un sel d'organoétain.

Toutefois la présence de ce sel métallique n'est pas sans présenter des inconvénients. Il peut être en effet à l'origine d'une stabilité insuffisante au stockage. Par ailleurs pour l'enrobage de médicaments, pour le revêtement d'articles en contact avec des aliments et des boissons tels que les bouchons de liège, l'homme de métier est à la recherche de compositions organopolysiloxanes pouvant réticuler en un élastomère silicone sans catalyseur métallique de durcissement.

Précisément un but de la présente invention est de proposer une dispersion aqueuse de silicone exempte de catalyseur de durcissement, et qui réticule convenablement en un élastomère après élimination de l'eau.

Un autre but de la présente invention est de proposer une dispersion aqueuse de silicone du type ci-dessus qui soit stable au stockage, qui réticule correctement et suffisamment rapidement en un élastomère par élimination de l'eau à température ambiante, l'élastomère formé conservant et même améliorant ses propriétés mécaniques lors de son vieillissement.

Un autre but de la présente invention est de proposer une dispersion aqueuse de silicone du type ci-dessus dont l'étape de maturation puisse être effectuée à une température peu élevée (20-60 °C).

Un autre but de la présente invention est de proposer une dispersion aqueuse de silicone du type ci-dessus conduisant à un élastomère présentant en outre une résistance à la flamme améliorée.

Un autre but de la présente invention est de proposer une dispersion aqueuse de silicone du type ci-dessus conduisant à un élastomère présentant une adhérence satisfaisante sur divers supports en particulier sur verre, béton et métaux (acier, aluminium).

Ces buts et d'autres sont atteints par la présente invention qui concerne en effet une dispersion aqueuse de silicone, réticulant en un élastomère par élimination de l'eau dans les conditions ambiantes, caractérisée en ce qu'elle comporte :

(A) - 100 parties d'une émulsion du type huile dans eau d'un $\alpha,\omega$-(dihydroxy)polydiorganosiloxane stabilisée par au moins un agent tensio-actif choisi parmi les agents tensio-actifs anioniques et non-ioniques et leurs mélanges,

(B) - 0,1 à 15 parties calculées en extraits secs, d'un organosiliconate alcalin ou alcalino-terreux en solution aqueuse,

(C) - 5 à 250 parties d'une charge minérale non siliceuse, ladite dispersion ayant un pH supérieur à 7, de préférence entre 8 et 13, et une teneur en extraits secs d'au moins 40 %.

Les $\alpha,\omega$-(dihydroxy)polydiorganosiloxane doivent avoir une viscosité d'au moins 100 mPa.s à 25 °C, de préférence d'au moins 50 000 mPa.s.

C'est en effet pour des viscosités supérieures à 50 000 mPa.s que l'on obtient un élastomère présentant un ensemble de propriétés mécaniques convenables, en particulier au niveau de la dureté Shore A et de l'allongement.

En outre plus la viscosité est élevée et plus les propriétés mécaniques se conservent lors du vieillissement de l'élastomère.

Les viscosités préférées pour la présente invention sont comprises entre 50 000 et 1 500 000 mPa.s à 25 °C.

Les radicaux organiques des $\alpha,\omega$-(dihydroxy)polydiorganopolysiloxanes sont des radicaux hydrocarbonés monovalents contenant jusqu'à 6 atomes de carbone, éventuellement substitué par des groupes cyano ou fluoro. Les substituants généralement utilisés du fait de leur disponibilité dans les produits industriels sont les radicaux méthyle, éthyle, propyle, phényle, vinyle et 3,3,3-trifluoropropyle. En général au moins 80 % en nombre de ces radicaux sont des radicaux méthyle.

Dans le cadre de la présente invention on préfère plus spécialement utiliser les $\alpha,\omega$-(dihydroxy)-polydiorganosiloxanes préparés par le procédé de polymérisation anionique décrit dans les brevets américains précités : US-A-2891 920 et surtout US-A-3 294 725 (cités comme référence). Le polymère obtenu est stabilisé anioniquement par un agent tensio-actif qui, conformément à l'enseignement de US-A3 294 725 est de préférence le sel d'un métal alcalin d'un acide hydrocarboné aromatique sulfonique, l'acide libre jouant également le rôle de catalyseur de polymérisation.

Le catalyseur et l'agent tensio-actif sont l'acide dodécylbenzènesulfonique et ses sels de métaux alcalins, en particulier son sel de sodium. On peut ajouter éventuellement d'autres agents tensio-actifs anioniques ou non-ioniques. Toutefois cet ajout n'est pas nécessaire car, conformément à l'enseignement de US-A-3 294 725, la quantité d'agent tensio-actif anionique résultant de la neutralisation de l'acide sulfonique est suffisante pour stabiliser l'émulsion de polymère. Cette quantité est généralement inférieure à 3 %, de préférence 1,5 % du poids de l'émulsion.

Ce procédé de polymérisation en émulsion est particulièrement intéressant car il permet d'obtenir directement l'émulsion (A). Par ailleurs ce procédé permet de pouvoir obtenir sans difficulté des émulsions (A) de $\alpha,\omega$-(dihydroxy)polydiorganosiloxane de très haute viscosité.

Pour préparer l'émulsion (A) on peut également partir d'$\alpha,\omega$-(dihydroxy)polydiorgosiloxane déjà polymérisé, puis le mettre en émulsion aqueuse en stabilisant les émulsions par un agent tensio-actif anionique et/ou non-ionique suivant un procédé bein connu de l'homme de métier et décrit en détail dans la littérature (voir par exemple les brevets FR-A-2 064 563, FR-A-2 094 322, FR-A-2 114 230 et EP-A-169 098).

Selon ce procédé, on mélange par simple agitation les polymères $\alpha,\omega$-(dihydroxy)polydiorganosiloxane avec l'agent tensio-actif anionique ou non-ionique, ce dernier pouvant être en solution aqueuse, à ajouter

3

ensuite si nécessaire de l'eau et à transformer l'ensemble en une émulsion fine et homogène par passage dans un broyeur classique à colloïdes.

Par la suite le broyat obtenu est dilué par une quantité d'eau appropriée et on obtient ainsi une émulsion (A) stabilisée par un agent tensio-actif anionique ou non-ionique stable au stockage.

La quantité d'agent tensio-actif anionique et non-ionique utilisable est celle utilisée couramment pour la mise en oeuvre du procédé de mise en émulsion, en particulier ceux décrits dans les brevets précités et dans le brevet US-A-2 891 920.

Dans le cadre de la présente invention les agents tensio-actifs anioniques préférés sont le sel d'un métal alcalin d'un acide hydrocarboné aromatique sulfonique et les agents tensio-actifs non-ioniques préférés sont les alkylphénols polyoxyéthylénés. Ces agents tensio-actifs non-ioniques sont bien entendu les mêmes que ceux que l'on peut ajouter éventuellement aux émulsions (A) obtenues par polymérisation en émulsion comme indiqué plus haut.

L'émulsion (A) préparée par polymérisation en émulsion ou par mise en émulsion du polymère silicone se présente sous la forme d'une émulsion huile dans l'eau et a, de préférence, une teneur en extrait sec supérieure à 45 % en poids.

Pour 100 parties d'émulsion (A), on incorpore de 0,1 à 15, de préférence de 0,5 à 5, parties, calculées en extraits secs, d'un organosiliconate (B) alcalin ou alcalino-terreux en solution aqueuse.

Ces organosiliconates alcalins ou alcalino-terreux sont des produits connus dont la plupart sont disponibles dans le commerce. Les plus courants sont les méthylsiliconates de sodium ou de potassium à environ 30 à 60 % d'extraits secs.

Les organosiliconates alcalins peuvent être préparés par exemple par hydrolyse ds organosilanes correspondants présentant 3 groupes hydrolysables, tels que des atomes d'halogènes des radicaux alcoxy, suivi d'une dissolution du produit obtenu dans une solution d'une base inorganique forte, dans des proportions telles qu'il y ait au moins un équivalent en base par atome de silicium (voir par exemple US-A-2 441 422, US-A-2 441 423 et US-A-2 507 200).

Un autre constituant de l'émulsion selon l'invention est l'addition de 5 à 250, de préférence de 10 à 200 parties d'une charge minérale semi-renforçante ou de bourrage (C).

Les charges (C) ont une granulométrie généralement comprise entre 0,001 et 300 $\mu$m et une surface BET inférieure à 100 m²/g.

Des exemples de charges (C) utilisables seules ou en mélange sont le noir de carbone, le dioxyde de titane, l'oxyde d'aluminium, l'alumine hydratée, la vermiculite expansée, la vermiculite non expansée, le carbonate de calcium, l'oxyde de zinc, le mica, le talc, l'oxyde de fer, le sulfate de baryum et la chaux éteinte.

Ces charges (C) sont introduites dans l'émulsion sous forme de poudre sèche par exemple par simple mélange.

Selon une variante de l'invention, on a découvert que si la charge (C) n'est sensiblement constituée que d'une charge choisie parmi l'alumine hydratée, la vermiculite expansée, la vermiculite non expansée selon une teneur de 5 à 250, préférence de 50 à 200 parties pour 100 parties d'émulsion (A) on obtient un élastomère ayant une résistance à la flamme particulièrement élevée qui ne peut pas être obtenue avec les autres catégories de charge (C) précitées, en particulier avec l'oxyde d'aluminium ou alumine non hydratée. On peut également incorporer des fibres céramiques ou d'aramide selon l'enseignement de EP-A-212 827.

Selon une variante, on peut incorporer en outre, pour 100 parties d'émulsion (A) un additif silicié (D) choisi parmi du silicate de soude (0,3 à 30 parties), et une charge siliceuse renforçante ou semi-renforçante (1 à 150 parties).

Ces charges siliceuses sont choisies parmi la silice colloïdale, les poudres de silice de combustion et de précipitation ou leur mélange. La silice de combustion est préférée. On peut toutefois utiliser également des charges siliceuses semi-renforçantes telles que des terres de diatomées, du quartz broyé.

Pour 100 parties d'émulsion (A), la somme des parties de (C) + (D) doit être inférieure à 300 parties.

Les poudres de silice de combustion et de précipitation sont bien connues, elles sont utilisées en particulier comme charges dans les compositions élastomère de silicone, vulcanisables à chaud en un caoutchouc de silicone. Ces poudres présentent une taille moyenne de particule généralement inférieure à 0,1 $\mu$m et une surface spécifique BET supérieure à 50 m²/g, de préférence comprise entre 150 et 350 m²/g.

L'incorporation dans l'émulsion (A) de cet additif silicié (D), par tout moyen convenable, en particulier par agitation, augmente considérablement la viscosité de l'émulsion (A) qui présente alors un caractère pâteux.

On a en effet trouvé, conformément à la présente invention que l'addition de cet additif silicié (D), est suffisante pour conférer à l'émulsion un caractère "thixotrope" plus ou moins marqué. L'émulsion, extraite

4

par exemple d'une cartouche de stockage, adhère sans s'écouler sur un substrat même vertical et durcit en élastomère par évaporation de l'eau à température ambiante. On peut également obtenir une émulsion non coulante en utilisant comme charge (C) du carbonate de calcium dont le diamètre particulaire moyen est inférieur à 0,1 $\mu$m. Bien entendu un léger chauffage (à 40-80 °C environ) de la composition pour accélérer l'évaporation de l'eau n'est pas exclu du cadre de l'invention.

Les dispersions aqueuses peuvent également une résine silicone hydroxylée (E).

Pour 100 parties d'émulsion (A), on peut incorporer en outre de 1 à 20, de préférence de 2 à 10 parties, calculées en extraits secs, d'une résine silicone hydroxylée (E).

La résine silicone hydroxylée (E) possède une teneur pondérale en groupe hydroxyle comprise entre 0,1 et 10 %, de préférence entre 1 et 6 %.

Cette résine (E) présente, par molécule, au moins deux motifs différents choisis parmi ceux de formules : $R_3SiO_{0,5}$ (motif M), $R_2SiO$ (motif D), $RSiO_{1,5}$ (motif T) et $SiO_2$ (motif Q).

Les motifs M, D, T et Q sont distribués de telle façon que le rapport molaire R/Si soit inférieur à 2, de préférence inférieur à 1,8, pour exclure les polydiorganosiloxanes linéaires.

Les radicaux R sont choisis parmi les radiciaux alkyle en $C_1$-$C_6$, vinyle, phéhyle et trifluoro-3,3,3 propyle.

Comme exemples de radicaux R alkyle on peut citer les radicaux méthyle, éthyle, isopropyle, tertiobutyle et n-hexyle.

Ces résines silicones sont des polymères organopolysiloxanes ramifiés bien connus dont les procédés de préparation sont décrits dans de très nombreux brevets.

Comme exemples de résines utilisables on peut citer les résines MQ, les résines MDQ, les résines TD et les résines MDT.

On peut utiliser les résines qui sont solides ou liquides à température ambiante. Ces résines peuvent être incorporées dans les émulsions aqueuses telles quelles, en solution dans un solvant organique ou une huile silicone, ou bien sous forme d'émulsions aqueuses.

Des émulsions aqueuses de résines silicones utilisables sont par exemple décrits dans les brevets US-A4 028 339, US-A-4 052 331, US-A-4 056 492, US-A-4 525 502 et US-A-4 717 599, cités comme référence.

Aux dispersions conformes à la présente invention peuvent être éventuellement ajoutés divers additifs permettant de modifier les propriétés de dispersions et des élastomères formés à partir des dispersions par élimination de l'eau. On peut incorporer en particulier un additif (F) choisi parmi les organotrialcoxysilanes, comme par exemple le vinyltriméthoxysilane, les silicates d'alkyle tels que le silicate de méthyle ou le silicate d'éthyle ou leur produit d'hydrolyse partielle, c'est-à-dire les polysilicates d'alkyle tels que le polysilicate de méthyle et le polysilicate d'éthyle à une teneur de 0,1 à 20 parties d'additif (F) pour 100 parties d'émulsions (A). L'additif (F) permet d'améliorer la cohésion du matériau élastomère obtenu après élimination de l'eau.

Les organotrialcoxysilanes et les silicates d'alkyle répondent de préférence à la formule générale :

$$R''_aSi(OR')_{4-a}$$

dans laquelle R' est un radical alkyle ayant de 1 à 4 atomes, R'' est R' ou vinyle et a est 1 ou 0.

Comme autres exemples d'additifs on peut citer les antifongiques, les antimousses ainsi que les agents thixotropants comme la carboxyméthylcellulose, la gomme xanthane et l'alcool polyvinylique.

Les dispersions selon l'invention peuvent être préparés de la façon suivante :

On part d'une émulsion (A) préparée soit par le procédé de polymérisation en émulsion et l'on dispose d'une émulsions stabilisée par un agent tensio-actif anionique et éventuellement non-ionique, soit par le procédé de mise en émulsion de l'α,ω-(dihydroxy)polydiorganosiloxane et l'on dispose d'une émulsion stablisée par un agent tensio-actif anionique et/ou non-ionique.

Pour préparer les dispersions selon l'invention il est recommandé d'ajouter à température ambiante à l'émulsion (A), en premier lieu l'organosiliconate (B), puis on règle, si besoin est, le pH du mélange à une valeur supérieure à 7 au moyen d'une base minérale ou organique. Comme base organique on peut utiliser les amines primaires telles que la diéthylamine. Cependant, selon un mode de réalisation préféré de l'invention, on règle le pH au moyen d'une quantité adaptée d'une base minérale introduite sous la forme d'une solution aqueuse choisie de préférence parmi les solutions d'hydroxydes alcalins et alcalino-terreux telles que la soude, la potasse, les solutions d'hydroxyde de calcium, l'hydroxyde de baryum et l'hydroxyde de magnésium. Toutefois les hydroxydes alcalino-terreux peuvent être introduits directement sous forme solide.

On ajoute les charges (C) et éventuelleent les charges siliceuses (D), la résine (E) telle quelle, ou en solution dans un solvant organique ou dans une huile silicone, ou bien sous forme d'une émulsion aqueuse et l'agent d'adhérence (F).

Comme huile silicone associée à la résine (E) on peut utiliser un polydiméthylsiloxane bloqué triméthylsilyle, de viscosité à 25 °C comprise entre 100 et 5000 mPa.s.

L'émulsion finale obtenue est homogénéisée puis dégazée et est ensuite conditionnée en emballage étanche à l'oxygène de l'air et à la vapeur d'eau.

Les constituants (A), (B), (C), et éventuellement (D), (E) et (F) sont mélangés en des quantités telles que l'émulsion finale présente une teneur en extrait sec supérieure à 40 %, de préférence supérieure à 60 %, mais généralement inférieure à 90 %. La zone pH préférée est comprise entre 8 et 13.

Les dispersions selon l'invention peuvent être utilisées comme peinture réticulable en couche mince. Elles présentent alors, de préférence, un extrait sec compris entre 40 et 70 %.

Pour déterminer la teneur en extrait sec on place 2 g de dispersion dans une coupelle de pesage en aluminium et on la chauffe une heure à 150 °C dans un four à circulation d'air. Après refroidissement on pèse à nouveau la coupelle et on détermine le pourcentage de matière restante sur les 2 g initiaux qui représente la teneur en extrait sec.

Selon une variante préférée, la dispersion selon l'invention après sa préparation subit une étape de maturation, à température ambiante, de quelques heures à quelques jours.

Cette étape de maturation consiste simplement à laisser reposer la dispersion à l'abri de l'oxygène de l'air avant son utilisation.

Les dispersions selon l'invention peuvent être utilisées pour la réalisation de joints élastomères silicone, en particulier pour le bâtiment.

Ces dispersions sont également utilisables pour l'enrobage de divers principes actifs pharmaceutiques ou phytosanitaires formulés sous une forme solide (pastilles, tablettes, pillules, etc .....), pour l'enduction de bouchons de liège utilisés pour obturer les bouteilles de vins et de spiritueux, pour réaliser des revêtements d'objets culinaires et, de façon générale, d'objets en contact avec des aliments (par exemple des moules à pain).

Les techniques connues d'enrobage sont utilisables en particulier les techniques d'enduction au pinceau et au trempé (par immersion), les techniques de pulvérisation, les techniques d'enrobage en lit fluidisé et les techniques d'enduction par immersion.

Pour les revêtements de bouchons de liège, une technique recommandée est la technique au trempé qui consiste à immerger les bouchons dans la dispersion qui mouille la surface du bouchon, puis à évaporer l'eau.

Le revêtement obtenu représente 20 à 50 mg d'élastomère pour 100 cm² de surface de bouchon. Cette couche facilite le glissement du bouchon dans le goulot de la bouteille lors de l'embouteillage et évite le "coulage", c'est-à-dire des fuites de liquide entre le goulot et le bouchon.

Un avantage spécifique tout à fait surprenant et inattendu des dispersions selon l'invention vis-à-vis des dispersions connues contenant un catalyseur métallique de durcissement, généralement un organo-étain, en particulier vis-à-vis des dispersions divulguées par EP-A-266 729 précité, est que les dispersions selon l'invention conduisent à des élastomères qui présentent une conservation et même une amélioration de leurs propriétés mécaniques au vieillissement normal ou accéléré. Par contre les élastomères issus des dispersions connues contenant un catalyseur métallique de durcissement présentent, de façon prévisible, une détérioration de l'ensemble de leurs propriétés mécaniques au cours de leur vieillissement normal ou accéléré.

Cet avantage spécifique des dispersions selon l'invention se traduit en particulier par une augmentation des résistances à la rupture et des allongements à la rupture qui vont dans le sens de l'obtention d'un élastomère de meilleure qualité au cours de son vieillissement, ce qui constitue un phénomène tout à fait inattendu pour l'homme de métier, d'autant plus que les dispersions selon l'invention présentent un temps de prise et une durée de maturation tout à fait acceptables et conduisent à des élastomères présentant des propriétés mécaniques finales analogues aux élastomères issus de ces dispersions connues et comportant un catalyseur métallique.

Dans ce qui suit ou ce qui précède, sauf mentions contraires, les pourcentages et parties sont en poids.

- EXEMPLE 1 :

On prépare une émulsion (A) par polymérisation en émulsion d'huile $\alpha,\omega$-(dihydroxy)-polydiméthylsiloxane de viscosité 100 mPa.s à 25 °C en présence d'acide dodécylbenzène sulfonique.

Quand la viscosité de l'huile atteint $1,5.10^6$ mPa.s à 25 °C, on arrête la polymérisation par neutralisation du catalyseur.

A 170 parties d'émulsion (A) à 58 % d'extraits secs on ajoute et on homogénéise à 25 °C, 7 parties d'une solution de méthylsiliconate de potassium à 40 % d'extrait sec.

Après 10 minutes d'homogénéisation, on ajoute 100 parties de CaCO₃ de granulométrie moyenne de 70 nanomètre.

La dispersion finale a une teneur en extrait sec de 73,2 % et présente un pH supérieur à 9.

Cette dispersion est homogénéisée pendent 30 minutes puis conditionnée sous un emballage étanche à l'oxygène de l'air et à la vapeur d'eau.

Après 5 jours de stockage, on étend à la râcle la dispersion suivant une pellicule (film) de 2 mm d'épaisseur qu'on laisse sécher pendant 7 jours à température ambiante (20 °C).

Sur un premier lot de pellicules séchées, on mesure les propriétés mécaniques moyennes suivantes :
- la dureté Shore A (DSA) selon la norme ASTM-D-2240,
- la résistance à la rupture (R/R) selon la norme AFNORT-T 46 002 correspondant à la norme ASTMD 412, en MPa,
- l'allongement à la rupture (A/R) en % selon la norme AFNOR-T 46 002,
- le module élastique (ME) à 100 % d'allongement selon la norme AFNOR-T 46 002, en MPa.

Sur un deuxième lot de pellicules séchées, on mesure les propriétés mécaniques après un vieillissement naturel des pellicules à température ambiante.

Les propriétés mécaniques obtenues sont rassemblées dans le tableau ci-après.

Il ressort du tableau qu'après 6 mois de vieillissement, l'élastomère conserve sensiblement les mêmes valeurs pour la DSA et le ME, et améliore nettement la R/R et le A/R.

Pour apprécier l'adhérence, on dépose sur un support en verre ou en béton, un cordon de dispersion aqueuse de 4 mm d'épaisseur. Après 12 jours on apprécie l'adhérence de l'élastomère formé en tirant manuellement sur le cordon.

L'adhérence est bonne sur les deux supports, le cordon n'ayant pu être décollé manuellement de son support.

- EXEMPLE 2 :

On répète exactement le mode opératoire de l'exemple 1 sauf qu'après l'addition du siliconate, on ajoute 7 parties en poids de vinyltriméthoxysilane. Le pH de la dispersion finale est de 11.

Les propriétés mécaniques sont rassemblées dans le tableau ci-après.

L'adhérence est bonne sur support en verre ou en béton.

- EXEMPLE 3 :

On répète exactement le mode opératoire de l'exemple 1 sauf qu'après l'addition de siliconate, on ajoute en outre 5 parties en poids d'hydroxyde de magnésium. Le pH de la dispersion finale est de 12.

Les propriétés mécaniques sont rassemblées dans le tableau ci-après.

L'adhérence est bonne sur support en verre ou en béton.

- EXEMPLE 4 :

On répète exactement le mode opératoire de l'exemple 1 sauf que :

a) - on part d'une émulsion (A) dont l'huile silicone présente une viscosité de $10^6$ mPa.s à 25 °C,

b) - on ajoute en outre 2 parties en poids de polysilicate d'éthyle (éthylsilicate 40®, commercialisé par UNION CARBIDE CORPORATION) et

c) - le deuxième lot de pellicules sont soumises à un vieillissement de 7 jours à 50 °C dans une étuve ventilée.

Les propriétés mécaniques obtenues sont rassemblées dans le tableau ci-après.

L'adhérence est bonne sur support en verre ou en béton.

Il ressort du tableau qu'après vieillissement accéléré les propriétés mécaniques se trouvent améliorées.

TABLEAU

| EXEMPLES | | 1 | 2 | 3 | 4 |
|---|---|---|---|---|---|
| DSA | 7 jours à 20 °C | 14 | 19 | 12 | 25 |
| | 6 mois à 20 °C | 12 | - | - | - |
| | 7 jours à 50 °C | - | - | - | 28 |
| R/R (MPa) | 7 jours à 20 °C | 0,33 | 1,15 | 0,63 | 0,45 |
| | 6 mois à 20 °C | 0,63 | - | - | - |
| | 7 jours à 50 °C | - | - | - | 1,08 |
| A/R (%) | 7 jours à 20 °C | 540 | 1 221 | 936 | 190 |
| | 6 mois à 20 °C | 980 | - | - | - |
| | 7 jours à 50 °C | - | - | - | 484 |
| M/E (MPa) | 7 jours à 20 °C | 0,19 | 0,26 | 0,22 | 0,96 |
| | 6 mois à 20 °C | 0,17 | - | - | - |
| | 7 jours à 50 °C | - | - | - | 0,44 |

## Revendications

1. - Dispersion aqueuse de silicone, réticulant en un élastomère par élimination de l'eau dans les conditions ambiantes, caractérisée en ce qu'elle comporte, en poids :
(A) - 100 parties en poids d'une émulsion du type huile dans eau d'un $\alpha,\omega$-(dihydroxy)polydiorganosiloxane stabilisée par au moins un agent tensio-actif choisi parmi les agents tensio-actifs anioniques et non-ioniques et leurs mélanges,
(B) - 0,1 à 15 parteis en poids, calculées en extraits secs, d'un organosiliconate alcalin ou alcalino-terreux en solution aqueuse,
(C) - 5 à 250 parties en poids d'une charge minérale non siliceuse,
ladite dispersion ayant un pH supérieur à 7 et une teneur en extrait sec d'au moins 40 %.

2. - Dispersion aqueuse de silicone selon la revendication 1, caractérisée en ce que l'émulsion (A) a une teneur en extrait sec d'au moins 45 % en poids.

3. - Dispersion aqueuse de silicone selon l'une quelconque des revendications précédentes, caractérisée en ce que la charge (C) est choisie parmi l'alumine hydratée, l'alumine, le carbonate de calcium, la vermiculite expansée, la vermiculite non expansée, le noir de carbone, l'oxyde de zinc, le dioxyde de titane, le mica, le talc, l'oxyde de fer, le sulfate de baryum et la chaux éteinte.

4. - Dispersion aqueuse de silicone selon la revendication 3, caractérisée en ce que le carbonate de calcium a un diamètre particulaire moyen inférieur à 0,1 $\mu$m.

5. - Dispersion aqueuse de silicone selon l'une quelconque des revendications précédentes, caractérisée en ce qu'elle comporte :
(A) : 100 parties de l'émulsion du type huile dans eau d'un $\alpha,\omega$-(dihydroxy)polydiorganosiloxane de viscosité à 25 °C comprise entre 50 000 et 1 500 000 mPa.s et est stabilisée par un agent tensio-actif choisi parmi un sel de métal alcalin d'un acide hydrocarboné aromatique sulfonique et les alkylphénols polyoxyéthylénés.
(B) : 0,5 à 5 parties d'un organosiliconate,
(C) : 50 à 200 parties d'une charge minérale,
ladite dispersion ayant un pH comprise entre 8 et 13 et une teneur en extraits secs d'au moins 60 %.

6. - Dispersion aqueuse de silicone selon l'une quelconque des revendications précédentes, caracterisée en ce qu'elle comporte en outre pour 100 parties d'une émulsion (A), un additif silicié (D) choisi parmi le silicate de soude (0,3 à 30 parties), et une charge siliceuse renforçante ou semi renforçante (1 à 150 parties), sous réserve que pour 100 parties de (A), la somme des parties de (C) + (D) soit inférieure à 300 parties.

7. - Dispersion aqueuse de silicone selon l'une quelconque des revendications précédentes, caractérisée en ce qu'elle comporte en outre, pour 100 parties d'émulsion (A), 1 à 20 parteis en poids d'une résine silicone hydroxylée (E) présentant, par molécule, au moins deux motifs différents choisis parmi ceux de formules : $R_3SiO_{0,5}$, $R_2SiO$, $RSiO_{1,5}$ et $SiO_2$, les radicaux R, identiques ou différents, étant choisis parmi les radicaux vinyle, phényle, trifluoro-3,3,3 propyle et les radicaux alkyle linéaires ou ramifiés ayant de 1 à 6 atomes de carbone inclus, ladite résine ayant une teneur pondérale en groupe hydroxyle comprise entre 0,1 et 10 %.

8. - Dispersion aqueuse de silicone selon l'une quelconque des revendications précédentes, caractérisée en ce qu'elle comporte en outre, pour 100 parties d'émulsion (A), de 0,01 à 20 parties d'additif (F) choisi parmi les organotrialcoxysilanes, les silicates d'alkyle et les polysilicates d'alkyle.

9. - Dispersion aqueuse de silicone selon l'une quelconque des revendications précédentes, caractérisée en ce qu'elle comporte en outre une solution aqueuse d'une base minérale choisie parmi les hydroxydes alcalines et alcalino-terreux.

10. - Dispeprsion selon la revendication 9, caractérisée en ce que les hydroxydes alcalino-terreux sont introduits directement sous une forme solide.

11. - Procédé de préparation d'une dispersion aqueuse de silicone telle que définie à l'une quelconque des revendications 1 à 10, caractérisée en ce que :

1) - on mélange l'émulsion (A), et l'organosiliconate (B),

2) - on ajoute la charge non siliceuse (C) et éventuellement les additifs siliciés (D), (E), (F) et la base minérale ou organique,

3) - on règle éventuellement le pH à une valeur supérieure à 8,5, la quantité d'eau totale utilisée étant telle que la teneur en matières solides de l'émulsion finale obtenue soit d'au moins 40 %.

12. - Utilisation d'une dispersion aqueuse telle que définie à l'une quelconque des revendications 1 à 10 pour l'enduction et l'enrobage de principes actifs pharmaceutiques ou phytosanitaires et pour la realisation de revêtements d'objets en contact avec des aliments.

13. - Utilisation selon la revendication 12, caractérisée en ce que lesdits objets en contact avec des aliments sont des bouchons de liège pour le conditionnement de vins et spiritueux.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 277 740 (DOW CORNING CORP.) <br> * page 2, lignes 1-8; exemples 1,2; revendications 1-5 * <br> --- | 1,2,5,6 ,11,12 | C 08 J 3/03 <br> C 08 L 83/04 <br> C 08 K 5/54 <br> C 09 D 183/04 |
| D,A | EP-A-0 266 729 (WACKER-CHEMIE GMBH) <br> * en entier * <br> --- | 1-3,5-9 ,11 | |
| A | US-A-4 618 645 (T. M. BAUMAN et al.) <br> * colonne 4, lignes 6-18; exemple 1; revendications * <br> --- | 1,2,5,6 ,9,11 | |
| D,A | EP-A-0 169 098 (RHONE-POULENC) <br> * page 1, lignes 4-9; page 17, ligne 23 - page 18, ligne 1; exemple; revendications 1,2 * <br> --- | 1,2,5,7 ,11,12 | |
| A | EP-A-0 202 494 (TORAY SILICONE CO. LTD.) <br> * colonne 8, ligne 44 - colonne 10, ligne 17; tableaux 1,2; colonne 17, lignes 1-8; revendications * <br> ----- | 1-5,8,9 ,11 | |

DOMAINES TECHNIQUES
RECHERCHES (Int. Cl.5)

C 08 J 3/00
C 08 K 5/00
C 08 L 83/00

Le présent rapport a été établi pour toutes les revendications

| Lieu de la recherche | Date d'achèvement de la recherche | Examinateur |
|---|---|---|
| BERLIN | 11-01-1990 | HOEPFNER W.W.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)